# EUROPEAN PATENT SPECIFICATION

(11) **EP 3 738 589 B1**
(45) Date of publication and mention of the grant of the patent: **08.11.2023**
(21) Application number: 20175085.8
(22) Date of filing: 15.05.2020
(51) Int. Cl.: A61K 31/198, A61K 31/375, A61K 31/728, A61K 45/06, A61P 1/02, A61P 11/04, A61P 11/12, A61P 11/14

(54) **A COMPOSITION FOR THE PREVENTION AND/OR TREATMENT OF OROPHARYNGEAL MUCOSA DISEASES**
ZUSAMMENSETZUNG ZUR VORBEUGUNG UND/ODER BEHANDLUNG VON ERKRANKUNGEN DER OROPHARYNGEALEN SCHLEIMHAUT
COMPOSITION POUR LA PRÉVENTION ET/OU LE TRAITEMENT DE MALADIES DE LA MUQUEUSE OROPHARYNGIENNE

(30) Priority: 17.05.2019 IT 201900002623
(43) Date of publication of application: 18.11.2020
(73) Proprietor: Neilos S.r.l., 80063 Piano di Sorrento (NA) (IT)
(72) Inventor: DI MAIO, Umberto, 80063 PIANO DI SORRENTO (NA) (IT)
(74) Representative: Studio Torta S.p.A.

(56) References cited:
- EP-A2- 2 251 016
- WO-A2-2004/080427
- US-A1- 2005 266 064
- A MOSLEHI ET AL: "N-acetyl cysteine for prevention of oral mucositis in hematopoietic SCT: a double-blind, randomized, placebo-controlled trial", BONE MARROW TRANSPLANTATION, vol. 49, no. 6, 10 March 2014 (2014-03-10) , pages 818-823, XP055662829, GB ISSN: 0268-3369, DOI: 10.1038/bmt.2014.34
- GIUSEPPE COLELLA ET AL: "A translational journey on the efficacy of hyaluronic acid in the prevention of oral mucositis: Results of a phase 2 trial", JOURNAL OF ORAL PATHOLOGY AND MEDICINE,, vol. 48, no. Supplement 1, 1 April 2019 (2019-04-01), page 46, XP009518534, ISSN: 1600-0714
- CHAITANYA N C S KMUTHUKRISHNAN ARAO K PPRIYANKA D R ET AL: "Oral mucositis severity assessment by supplementation of high dose ascorbic acid during chemo and/or radiotherapy of oro-pharyngeal cancers - A pilot project", INDIAN JOURNAL OF PHARMACEUTICAL EDUCATION AND RESEARCH, ASSOCIATION OF PHARMACEUTICAL TEACHERS OF INDIA IND, IN, vol. 52, no. 3, 1 July 2018 (2018-07-01), pages 532-539, XP009518533, ISSN: 0019-5464, DOI: 10.5530/IJPER.52.3.61

## Description

### CROSS-REFERENCE TO RELATED APPLICATIONS

This patent application claims priority from Italian patent application no. 102019000002623 filed on 17/05/2019.

### TECHNICAL SECTOR

The present invention relates to a composition comprising an association of N-acetylcysteine, hyaluronic acid and/or its salts and *Vitamin C* as defined in claim 1 for the use in the prevention and/or treatment of the oropharyngeal mucosa diseases. Said composition is particularly effective thanks to the synergistic action of its components.

### STATE OF THE PRIOR ART

WO2004/080427 discloses the use of vitamin C to treat pathological conditions of the mucosa of the lower and upper respiratory tract such as oral sores, sore throat, pharyngeal inflammation, etc...

A MOSLEHI ET AL: "N-acetyl cysteine for prevention of oral mucositis in hematopoietic SCT: a double-blind, randomized, placebo-controlled trial", BONE MARROW TRANSPLANTATION, vol. 49, no. 6, 10 March 2014 (2014-03-10), pages 818-823, discloses that NAC reduces the incidence and grade of oral mucositis caused by chemotherapy.

GIUSEPPE COLELLA ET AL: "A translational journey on the efficacy of hyaluronic acid in the prevention of oral mucositis: Results of a phase 2 trial", JOURNAL OF ORAL PATHOLOGY AND MEDICINE, vol. 48, no. Supplement 1, 1 April 2019 (2019-04-01), page 46, discloses that hyaluronic acid reduces the risk of developing oral mucositis in patients undergoing chemotherapy.

CHAITANYA N C S KMUTHUKRISHNAN ARAO K PPRIYANKA D R ET AL: "Oral mucositis severity assessment by supplementation of high dose ascorbic acid during chemo and/or radiotherapy of oro-pharyngeal cancers - A pilot project", INDIAN JOURNAL OF PHARMACEUTICAL EDUCATION AND RESEARCH, ASSOCIATION OF PHARMACEUTICAL TEACHERS OF INDIA IND, vol. 52, no. 3, 1 July 2018 (2018-07-01), pages 532-539, discloses that vitamin C alleviates the severity of oral mucositis in patients with cancer.

### THE ORAL CAVITY

The oral cavity, or buccal cavity, is the first portion of the feeding channel that is placed at the head where it communicates with the outside environment by the buccal opening, delimited by a free border called lip. The mouth lips are defined and subdivided into upper and lower lip. Lips are covered with the skin and are composed of muscles, a sub-mucous tunic, a mucous tunic, glands, blood vessels and nerves. They can be divided into three parts: skin part, vermilion border, mucous part. Inside the mouth we can distinguish 2 parts communicating with each other:
- the vestibule;
- the buccal cavity (properly speaking).

The vestibule is a horseshoe-shaped slit placed between the lips at the front, the cheeks laterally and the alveolar dental arch at the back. The buccal cavity is comprised between the mouth vestibule and the isthmus of the fauces. The roof of the oral cavity consists of:
- Hard palate: it results from the fusion of the two maxilla palatine processes and at the back from the fusion of the two horizontal processes of the palatine bones. At the front, a small contribution is given by the nose-front spur.
- Soft palate: it is a muscular lamina that is attached at the back to the hard palate which is for closing the nasopharynx while swallowing; in practice the soft palate is normally oblique, whereas it becomes horizontal while swallowing by closing the nasopharynx.

The soft palate has:
- An upper face directed towards the pharynx and it contributes to form the floor of the nasal cavities;
- A buccal lower face, that is smooth and concave, is crossed in the middle by the soft palate
   raphe;
- Two side margins that enter into the medial lamina of the pterigoyd processes of the sphenoid bone;
- A lower free margin which has an appendix in the middle, the uvula.

At the sides of the uvula two muscles rise:
- The palatoglossus at the front which fixes at the side and back margins of the tongue delimiting the 1° palatine arch (the palate-glossal arch).
- The pharyngeal palate which fixes at the side margins of the oropharynx and constitutes the 2° arch (the palate-pharynx).

Between the 1° and the 2° palatine arch there is the palatine tonsil (paired), an aggregate of lymphoid tissue covered with mucous membrane. The surface of these glands penetrates into the lymphoid aggregates and forms crypts. Palatine arches and palatine tonsils form the side wall of the isthmus of fauces.

The palate muscular apparatus consists of the following muscles:
- Palatoglossal muscle;
- Palatopharyngeal muscle;
- Levator veli palatini muscle;
- Tensor veli palatini muscle which enters into the pterygoid hook-shaped process;
- The uvula muscle.

The floors of the oral cavity, as regards muscles, are formed by the mylohyoid muscle (it is hammock-shaped and connects the hyoid bone to the jaw arch) that is also the roof of the suprahyoid region. The sub-mandibular gland is under the mylohyoid muscle however it has a hook which also extends over the mylohyoid muscle and we can say that, to a minimal extent, this gland is also an oral cavity gland. From a musculocutaneous perspective, the floor 30 of the oral cavity is given by the tongue and the sublingual cleft (with lifted tongue).

The sublingual cleft is the horseshoe-shaped part between the lower dental arch until two palatine arches. In the middle of the sublingual cleft there is the tongue frenulum (connecting the sublingual cleft to the lower part of the tongue) at the two sides of which there is a relief, the sublingual caruncle (where the ducts 35 of the submandibular and sublingual glands outflow). Each caruncle goes outward into a relief, the sublingual fold (a prominence under which the sublingual gland lies).

### THE PHARYNX

The pharynx is a channel putting the throat in communication with the esophagus. It is a muscular-mucous structure, it represents both the first tract of the digestive tube - in fact it receives the food bolus from the mouth by swallowing - and a part of the upper airways: air coming from the nose is introduced into the pharynx, and from the pharynx into the larynx. Both the feeding channel and the airways thus flow into the pharynx and then continue respectively into the esophagus and the larynx. This channel, which is 15-centimetre-long, passes behind the nose cavities, the mouth and the larynx and extends vertically from the skull base to the sixth cervical vertebrae. It has a funnel-like shape: it is larger in the upper part, it narrows as it goes downward gradually assuming a tubular 15 aspect. The pharynx has two main functions:
- as it is the first tract of the digesting tube, the pharynx puts the mouth into communication with the esophagus allowing for the passage of the food bolus by swallowing;
- as it is part of the upper airways, the pharynx allows for the passage of air from the nose cavities to the larynx.

The human pharynx is conventionally divided into three sections: nasopharynx, oropharynx and laryngopharynx. The oropharynx is in particular the continuing pharynx region, and it extends from the plane of the upper surface of the soft palate to the plane of the upper surface of the hyoid bone (or floor of vallecula). It includes the base of the tongue, the lower (front) surface of the soft palate and the uvula, the front and back tonsillar pillars. There is no vault of the oropharynx in the back portion while at the front it consists of the lower surface of the soft palate. Its upper and lower surfaces are covered with mucous membrane, but the upper surface has a respiratory epithelium, while the lower one has a non-keratinized layered epithelium. Several glands are present into the mucous membrane constituting the greatest portion of the thickness of the soft palate, while the muscular component is placed more in depth; lymphoid aggregates are rather frequent. The anterior third of the soft palate is the least mobile as it mainly consists of palatine aponeurosis joining it to the hard palate and not of muscles; the palatine aponeurosis in turn consists in the extension of tendons of the tensor veli palatini muscle. The posterior end of the soft palate goes down into a drop-shaped process called uvula, also covered with mucous membrane and innerly consisting of the small uvula muscle. The side walls of the oropharynx consist of palatopharyngeal arches at the back and palatoglossal arches at the front. The palatoglossal arches originate from the soft palate and continue downward and frontward until the root of the tongue, they contain the palatoglossal muscle while the palatopharyngeal arches pass from the back portion of the soft palate to the pharynx wall and contain the palatopharyngeal muscle. A recess is formed between the two ipsilateral arches, the tonsillar fossa, where the palatine tonsil is accommodated. The back wall of the oropharynx upperly continues with the nasopharynx wall and it is at C2-C3 level.

### EXAMPLES OF OROPHARYNGEAL CAVITY DISEASES

Depending on the area, the oral cavity or the pharynx or other structures, it is possible to distinguish different pathological conditions characterized by ulcers, inflammations or infections.

### ORAL CAVITY ULCERS

Aphtous ulcers, also known as "aphtous stomatitis", are the more frequent ulcerative lesions of the oral cavity and can be considered within the frame of the auto-immune diseases targeting the entire epithelium of the oral mucous membrane. Such ulcerative lesions are often defined as "recurrent" in that in many instances they recur periodically. Up to 25-30% of the world population suffers from it within a rather wide range of age, even though the incidence during childhood and adolescence is twofold compared with the adult population (1.5% against 0.85%).

In some patients recurrent oral aphtous ulcers arise as an isolated disease while in other instances they can be the first sign of a more complicated pathological condition involving the gastrointestinal tract and other apparatuses. Aphtous ulcers appear as erosions with oval or round clean margins covered with a white or yellowish pseudo-membrane surrounded by an erythematous halo. Ulcers can be very painful and interfere with phonation and feeding. Sometimes the prodrome symptom consists in a burning sensation at the mucous membrane where, at a later time, ulcers will form. A feature of aphtous ulcers is their preference for non-keratinized and mobile mucous membrane. The most affected areas in a decreasing order are labial mucosa, cheek mucosa, lingual belly or margin, oral floor, soft palate and oropharynx. Aphtous ulcers are classified depending on their size, duration and presence or absence of scars after healing.

Minor sores (Mikulics ulcers) account for 75-85% of the aphtous ulcers and appear as lesions with a diameter lower than 1 cm, with a duration between 7 and 14 5 days and heal without leaving scars.

Major sores (Sutton disease) comprise about 10-15% of the cases. Ulcers have a diameter higher than 1 cm, are deep, more painful than minor ulcers, they take 6 weeks or more to heal and often leave scars.

Dysphagia and fever are frequently present. 10 Herpetiform sores account for 5-10% of oral ulcers and are so called in that they appear as several small ulcers with a diameter of 1-3 mm and look like herpes virus ulcers. Such lesions heal within 10-14 days without leaving scars.

### MUCOSITIS

It is an inflammation of the mucous membrane of the mouth and pharynx. It represents one of the most common side-effects of the anti-tumoral therapeutics (radiation therapy and chemotherapy), which can alter the integrity of the oropharyngeal tissues. It is a multifactorial disease defined as an epithelial thinning associated to a deep erythema, ulcer, pain, bleeding and increased risk of contracting an infection. At the beginning the mucous membrane is atrophic, followed by a severe erythema resulting in ulcers; the most affected areas are non-keratinized (oral floor, cheek mucosa, labial mucosa and tongue). Patients suffer from a burning feeling, oral cavity bleeding, pain such that they cannot eat, swallow and even speak. The inflammation process further compromises the barrier function of the oral mucous membrane and increases the risk of infections involving the soft tissues of the mouth.

### LEUKOPLAKIA

It is a typical lesion of the oral cavity which mainly involves the surface of the tongue and the inner mucous membranes of the lips. It forms as a result of an exceeding and abnormal keratinization of the epithelium and in some instances of a dystrophic thickening of some outer layers and of the spinous layer of the epithelium. It can be recognised as it forms whitish plaques that are often responsible for taste alteration and ailments inside the mouth. It does not always represent a lesion indicating a malignant neoplasm, however this does not mean that it can be disregarded. It can therefore be considered as a pre-cancerous lesion, as it is visibly different from the normal oral mucous membrane.

### PHARYNGITIS (FARINGOTONSILLITIS)

In common language, pharyngitis is better known as sore throat, a symptom which characterizes several pathologic circumstances. Pharyngitis can arise in an acute (sudden) manner and can heal in a few days, or can become chronic and last for long periods. In the latter case we talk about chronic pharyngitis. It is an inflammatory process regarding pharynx, uvula and tonsils, which is generally transmitted by direct contact with respiratory secretions. It is more frequent in paediatric age (5-15 years old), and, though it is sometimes self-limiting, the swelling of the parts involved can cause a reduced patency of the airways, or, in any case, hinder the ingestion of proper amounts of liquid with resulting dehydration. The infection can be due to viruses (such as Epstein-Barr) and bacteria, in particular Group A beta haemolytic Streptococcus pyogenes is the most frequent among children, as well as Micoplasma pneumoniae, Clamidia pneumoniae can be found in adults and children. Forms that are transmitted by sexual contact and supported by Neisseria gonorrhoeae and by Corynebacterium dyphtheriae (a form reduced by vaccine use) must further be taken into consideration.

### COUGH

From a physiological perspective, the coughing reflex is in itself an important defence as it is able to promote removal of the particulate which might contain allergens or pathogen microorganisms. In normal conditions cough plays a primary role in protecting the airways and the pulmonary parenchyma. When such process increases in frequency and intensity due to different factors it does no longer play a beneficial role and becomes a symptom for patients requiring medical advice. Cough is normally classified into acute, sub-acute and chronic cough. In the first case it lasts maximum 3 weeks and for the majority of patients it is due to infections of the upper respiratory tract, acute bronchitis or tracheobronchitis due to bacterial or viral infections. This type of cough usually clears up in a couple of weeks together with the complete resolution of the infection.

Sub-acute cough lasts between 3 and 8 weeks and it is generally related to specific infections (such as M. pneumoniae), where, a greater bronchial response can last longer even after resolution of the inflammation. Chronic and persistent cough is instead usually due to other causes such as cigarette smoking or use of ACE inhibitors, asthma or gastroesophageal reflux disease, cystic fibrosis.

### DYSPHONIA

"Dysphonia" is the medical term used to indicate a general qualitative and/or quantitative, temporary or long-lasting voice alteration, having structural or functional origin of one of the organs involved in the spoken phonation. Such alteration mainly means a difficulty in controlling the intonation, the voice timbre, the volume or the voice quality. Dysphonia can be associated to pain or ailment while speaking. The voice disorders can have different origins:
- ORGANIC: characterized by morphological and neuromuscular alteration of the structures involved in phonation. Often organic dysphonia is caused by laryngitis, neoplasms affecting lungs, pharynx, surgical traumas, endocrine diseases, use of corticosteroids by inhalation;
- FUNCTIONAL: consisting in an excess or deficit of the phonatory function, in cases of voice abuse, weakening of the phonic muscle, musculotensive alterations affecting the larynx.

There are also cases of PHONOASTHENIA, wherein a reduction in the intensity of the voice emission occurs, leading to a weak and strained voice.

### HOARSENESS

Hoarseness (often considered as a symptom of Dysphonia) is an abnormal change of the voice, sounding raspy and harsh, often flickering and associated with cough, sore throat, difficulty in swallowing and in breathing. The typical lowering in pitch, gradual or sudden, is often associated to an inflammatory component involving the airways or to a bacterial or viral infectious component. Beside the inflammatory and infectious causes, there are other factors that may contribute to the onset of the disorder such as neuromuscular alterations, neoplasms of several respiratory structures, gastroesophageal reflux, allergies, chronic cough, toxic substance inhalation, alcohol and tobacco consumption, systemic diseases.

### EPIGLOTTITIS

It deals with an inflammation of the epiglottis, resulting from a viral or bacterial infection, which determines the organ swelling with a possible obstruction of airways.

It is mainly caused by H. influenzae type-b, as well as streptococci, staphylococci, or thermal trauma. It exhibits ear pain (in adults), dysphonia, while fever is absent in 50% of cases and can develop at a later stage. Antibiotic treatment is used when bacteria are causing the disease, while intubation may be required in case of a severe airway obstruction 10.

### LARYNGITIS

It is an inflammation of the larynx which exhibits aphonia and hoarseness, mainly caused by viruses, but until 10% of cases also by bacteria (including streptococci and dyphtheriae). Non-infective causes can be tumours, thermal or caustic traumas, gastroesophageal reflux disease (GERD). Laryngitis has symptoms lasting 3-4 days and, unless bacteria are present, no antibiotic is used.

### Detailed description of the invention

The inventors of the present invention have now surprisingly discovered that the association of N-acetylcysteine, hyaluronic acid and/or its salts and Vitamin C, represents an effective alternative to commercially available products. Moreover, the association according to the present invention results into an unpredictable synergistic effect of its components.

### N-acetylcysteine

N-acetyl-L-cysteine (NAC) is the acetylated form of L-cysteine amino acid and it is presently employed in several disorders including: cystic fibrosis, paracetamol poisoning, chronic obstructive pulmonary disease, chronic bronchitis, doxorubicin-induced cardiotoxicity, HIV, heavy metal toxicity, neurological/psychiatric disorders.

NAC is a pro-drug and glutathione precursor which is a known antioxidant; furthermore, such molecule has an anti-inflammatory, antioxidant and antimicrobial activity.

In relation to the antioxidant activity, NAC interacts with the electrophilic groups of free radicals by thiol groups of its molecule. Its antioxidant activity is particularly rapid with respect to the hydroxyl radical (·OH), nitrogen dioxide (·NO2) and carbon trioxide (CO3-), while it does not directly interact with the nitric oxide but with its protonated form (HNO). Furthermore, NAC is able to chelate metal ions such as copper and iron as well as cadmium, mercury and lead 5 by complexation with the free thiol groups.

N-acetylcysteine can also act as an indirect antioxidant by increasing the intracellular concentration of GSH, the most important endogenous antioxidant. *In vitro* and *in vivo* studies have proved the antioxidant effectiveness of NAC towards several oxidative "insults" of the oral cavity such as blue light, fluoride exposure, hydrogen peroxide, nitric oxide, lipopolysaccharides etcetera. Regarding the anti-inflammatory activity NAC is able to reduce NFkB activity blocking translocation and activation avoiding I-kB degradation.

In oral inflammations NAC prevents the lipopolysaccharide-induced expression of citokines such as IL-1 beta, IL-6, IL-8, tumor necrosis factor (TNF).

Despite NAC is not an antibiotic, its effectiveness in reducing biofilm formation induced by a wide range of microorganisms has been proved.

For example scientific studies prove the N-acetylcysteine action towardsEnterococcus *faecalis* that is one of the most important opportunistic pathogens of the oral cavity, 20 as well as towards other bacteria such as *S.aureus, S. epidermidis, E. coli, P. aeruginosa* ecc.

In addition to the above-mentioned activities, it is widely known in literature the NAC mucolytic-fluidifying action; the action mechanism consists in reducing disulfide bridges of proteins in the mucus: this allows reducing viscosity and eases removal thereof. NAC action in treating chronic obstructive diseases has been widely demonstrated in the scientific literature; during HIACE study it was observed that in patients suffering from BPCO and treated with NAC an improvement in the forced expiratory flow passing from 25% to 75% and a reduction in exacerbations (from 1.71 times a year to 0.96) occur. Even another series of studies suggests the effectiveness of using NAC for treating BPCO: in this case patients being treated for six months with this molecule have shown a significant increase in FEV1 (Forced Expiratory Volume at the first second) and a maximum expiratory flow, while another study showed an increase in FEV1 from 25% to 30% after a short 4-week-therapy. Even exacerbations of the disease are reduced by 0.07 exacerbation/month and the days of discomfort are reduced by 0.56 days/month, which allows to reduce hospitalizations due to BPCO by 30%. As previously mentioned, N-acetylcysteine proves effective towards BPCO not only due to the mucolytic effect, but also to the anti-inflammatory one. NAC in fact is able to affect several factors involved in the inflammatory process, reducing the chemiotactic capacity of the patients mucus after 10-month-treatment and modulating the inflammatory response after 10-week-administration.

Furthermore, a meta-analysis has demonstrated the benefits of the prolonged use of N-acetylcysteine for the treatment and prevention of chronic bronchitis, proving capable of preventing acute exacerbations of this disease. For these reasons, N-acetyl-cysteine is applied for treating different diseases affecting the lower respiratory tract, such as pulmonary emphysema, bronchitis, amyloidosis, BPCO. For the purposes of the present invention NAC represents the component capable of providing immediate antioxidant action and a direct fluidifying activity on the oropharyngeal mucous membrane acting directly on the concerned area more effectively than standard treatments.

### Hyaluronic acid

The hyaluronic acid is an important component of the synovial fluid and of the extracellular matrix. In chemistry it is a natural polymer composed of alternate residues of glucoronic acid and n-acetyl-d-glucosamine and belongs to the class of substances known as glycosaminoglycans (GAGs). Within this class of compounds it is the structurally simplest compound, the only one that is not covalently linked to a protein and non-sulfated *core.* Fibroblasts are the main cells releasing hyaluronic acid into the extracellular matrix.

It is involved in important physiological processes: wound repair and regeneration, morphogenesis and structural organisation of the matrix thereof. The biological function of the hyaluronic acid is strictly related to its hydrophilic and hydrodynamic properties, enabling it to retain water and hence play an important structural role within the cells.

The molar mass can be up to 107 Da and, thanks to its viscoelastic and rheological properties it is an interesting component to be used for different medical applications. High- or low-molecular weight hyaluronic acid can be used. Generally speaking the low-PM HA has a cicatrizing and stimulating action in tissue repair, while the high-molecular weight one mainly has a protective barrier activity as it does not trigger any physiological, metabolic, immunologic process.

The topic application of hyaluronic acid allows for example to treat oral mucosa ulcers with a quick remission of symptoms thanks to its well-recognised anti-inflammatory properties.

It is used by injecting it intraarticularly in patients with a knee osteoarthritis, it is employed in ophthalmic preparations for ocular administration especially in eyewashes for the dry eye syndrome due to its lubricant action.

In the gastrointestinal field, recently various studies and formulations existing on the market demonstrate the use thereof to avoid gastroesophageal reflux- and hyperacidity-induced damages.

In relation to the present invention, the hyaluronic acid is particularly interesting for its protection and moisturising action on the mucous membranes of the oropharyngeal cavity. Furthermore its local inflammatory action allows rapidly relieving the symptoms related to the irritative state of the mucous membranes involved caused by problems of oxidative, inflammatory, bacterial or viral origin. It is known in the literature the compound ability to act on prostaglandins, metalloproteinases and other inflammatory mediators. The hyaluronic acid and its salts represent effective bio-active principles for preparing pharmaceutical, nutraceutical, dermocosmetic products or medical devices for the treatment of oropharyngeal cavity diseases by virtue of their moisturising, protective and regenerating action of the tissues of mucous membranes.

### Vitamin C

Vitamin C (or ascorbic acid) is a six-carbon atom lactone which is synthetized by glucose in the liver of different mammals, though not in man and other species which do not have the gulonolactone oxidase enzyme that is crucial for the synthesis of the ascorbic acid precursor (2-keto-I-gulonolactone).

When a person does not take up sufficient ascorbic acid through diet a deficiency status arises under a specific syndrome known as scurvy disease which may lead to problems such as blood vessel fragility, connective tissue damages, tiredness, etcetera. In addition to lack of dietary uptake, conditions such as alcoholism, age, kidney disorders, haemodialysis, mental problems and others, represent risk factors for reducing endogenous levels of vitamin C.

The minimum daily dose capable of preventing onset of the scurvy disease is 10 mg while the recommended daily dose is 80 mg (of which 75 mg for women and 90 mg for men).

The uptake of the molecule depends on a facilitated diffusion mechanism by means of a specific carrier whose saturation and *down regulation* determine plasmatic concentrations of vitamin C.

The accumulation of vitamin C can hardly occur as the exceeding amount for the body is removed through urine, however it may result into disorders such as nausea, diarrhoea, headache.

Vitamin C is an electron-donor and hence a reducing agent that oxidizes instead of other biological substances such as lipids, proteins, nucleic acids whose oxidation is involved in the development of several pathological conditions.

Being a hydrophilic molecule vitamin C can act both inside and outside cells contrasting the action of several free radicals such as nitrogen, superoxide radical, hydrogen peroxide, radical hydroxyl and singlet oxygen.

Several scientific studies have proved the biological importance of vitamin C and endogenous concentrations thereof in pathological conditions such as cardiovascular diseases and cancer or in specific populations such as people with diabetes or smokers.

The wide scientific literature on the molecule makes it a key ingredient for developing topic formulations in the oropharyngeal cavity especially in those conditions characterized by oxidative stress and inflammation.

Therefore, the association of the present invention allows to simultaneously obtain an antioxidant effect, anti-inflammatory effect and protection moisturising effect with respect to the oropharyngeal tract mucous membranes.

In fact, NAC is a known antioxidant acting both through an indirect and direct mechanism and it is further provided with anti-inflammatory and antimicrobial activity; the hyaluronic acid according to its PM plays a protective activity with a barrier effect, anti-inflammatory activity and cicatrisation-stimulating activity promoting healing of lesions or micro-lesions that may arise in case of oropharyngeal cavity diseases.

Finally, vitamin C contributes to increase the antioxidant effect of the final preparation.

### OBJECT OF THE INVENTION

It is an object of the present invention a composition comprising the association of extract of N-acetylcysteine, hyaluronic acid and/or a salt thereof and at least a source of Vitamin C in a mixture as defined in claim 1 with one or more pharmaceutically acceptable suitable carriers, for use in the prevention and/or treatment of the oropharyngeal mucosa diseases. NAC is present in an amount between 0.5 mg and 9000 mg, preferably between 1 mg and 7000 mg, still more preferably between 5 mg and 6000 mg. The hyaluronic acid is present in an amount between 0.5 mg and 5000 mg, preferably between 1 mg and 3000 mg, still more preferably between 5 mg and 2000 mg. Vitamin C is present in an amount between 0.5 mg and 5000 mg, preferably between 1 mg and 3000 mg, still more preferably between 5 mg and 2000 mg.

Pharmaceutically acceptable suitable carriers are those commonly known to the skilled in the art for preparing pharmaceutical compositions for oral administration (such as pastilles, candies, powders, granulates, tablets, solutions, suspensions, etcetera) and for topic application (such as creams, emulsions, gel, pastes, sprays, aerosols, sticks). For exemplary and non-limiting purposes, such pharmaceutical acceptable carriers may consist of diluents (such as biphasic calcium phosphate, lactose, microcrystalline cellulose and cellulose derivatives), thickeners (such as rubbers, hydroxypropyl methylcellulose and other cellulose derivatives), sweeteners (such as sorbitols, mannitol and other polyols, acesulfame K, aspartame, ciclammates, saccharine, sucralose), lubricants (such as magnesium stearate, stearic acid, waxes), dispersants, surfactants (such as sodium lauryl sulphate and polysorbates), flavouring agents, absorbing agents (such as silica gel, talc, starch, bentonite, kaolin), glidants and anti-adherents (such as talc, colloidal silica, cornstarch, silica dioxide), dyes (such as iron oxide), opacifiers (such as titanium oxide), antioxidants, binders (such as rubbers, starch, gelatine, cellulose derivatives, saccharose, sodium alginate) disgregating agents (starch, microcrystalline cellulose, alginic acid, crospovidone), plastifiers (such as ethyl cellulose and other cellulose derivatives, acrylates and metacrylates, glycerol and sorbitol), preservatives (such as parabens, sulfur dioxide ), viscosizing agents, emulsifiers, humectants, wetting agents, chelators, and mixtures thereof.

The composition object of the present invention can be incorporated in a medical device, a food supplement, a cosmetics, a nutraceutical, dietetic and nutritional composition, a food product, a beverage, a nutraceutical, a medicament, a medicated food, a pharmaceutical composition or a food for special medical purposes.

The composition object of the present invention is preferably a solid, semi-solid or liquid pharmaceutical composition for oral or topic use. In one embodiment, the composition object of the present invention is in the form of pastille, candy, powder, mouth dissolving powder, granulate, hard capsule, soft-gel capsule, tablet, or sachet. In another embodiment, the composition object of the present invention is in the form of solution, suspension or emulsion. In a further embodiment, the composition object of the present invention is in the form of spray, aerosol, stick, paste, gel, emulsion, cream for topic use.

The compositions of the present invention are particularly effective in the prevention and/or treatment of oropharyngeal mucosa disease thanks to the synergistic action of its components.

The composition object of the present invention exhibits a valid synergistic effect, in particular, when NAC is in an amount between 0.5 mg and 9000 mg, the hyaluronic acid and a salt thereof is in an amount between 0.5 mg and 5000 mg and, Vitamin C is in an amount between 0.5 mg and 5000 mg.

More in particular, the composition object of the present invention exhibits a valid synergistic effect when NAC is in an amount between 1 mg and 7000 mg, the hyaluronic acid is in an amount between 1 mg and 3000 mg and, Vitamin C is in an amount between 1 mg and 3000 mg.

It is therefore an object of the present invention a composition comprising the association of N-acetylcysteine, hyaluronic acid and/or its salts and Vitamin C in a mixture as defined in claim 1 with one or more pharmaceutically acceptable carriers for use in the prevention and/or treatment of oropharyngeal mucosa diseases.

Without being bound to the specific theory, the inventors believe that the effect of the association of N-acetylcysteine, hyaluronic acid and/or its salts and Vitamin C according to the present invention, derives from the following activities of the components of the association.

### Antioxidant activity

The effectiveness of the composition object of the present invention was assessed according to experimental protocols known to the skilled in the art.

In particular, in order to assess the antioxidant activity of the composition of the present invention, *in vitro* and/or *in vivo* essays that are known in the scientific literature can be employed. More in particular, in order to assess the antioxidant activity of the composition of the present invention, which is particularly interesting for the prevention and/or the treatment of the oropharyngeal cavity diseases, there are suitable *in vitro* essays such as: DPPH test, radical *scavenging* activity on the nitric oxide or on the nitric peroxide radical, TEAC test (Total radical-trapping antioxidant parameter), FRAP (Ferric reducing-antioxidant power), HORAC (Hydroxyl radical averting capacity), ORAC (Oxygen radical absorbance capacity) and the like.

In order to assess the antioxidant activity of the composition of the present invention, there are also suitable *in vivo* essays consisting in determining in animals the levels of endogenous substances such as, for example, reduced glutathione, glutathione peroxidase, glutathione S-transferase, superoxide dismutase and catalase, after administrating the concerned active principles. Even measuring the lipid peroxidation is a valid method for estimating the antioxidant activity of the present composition.

### Anti-inflammatory activity

In order to assess the anti-inflammatory effectiveness of the composition of the present invention, which is particularly interesting for the prevention and/or the treatment of the oropharyngeal cavity diseases, *in vitro* essays are suitable as essays that assess the capacity to inhibit the release of inflammatory cytokines as IL-1, IL-6 and TNF-α and to inhibit the expression of enzymes as COX-2 and IL-1β- induced metalloprotease-13 in primary cultures of human cells .

### Filmogen activity

In order to assess the filmogen effectiveness of the composition of the present invention, which is particularly interesting for the prevention and/or the treatment of the oropharyngeal cavity diseases, in vitro essays are suitable such as those determining tensile strength, shear stress, adhesion weight method, fluorescent probe method, flow channel method, mechanical spectroscopy methods, falling liquid film method, viscosimeter test, adhesion number, in vitro studies of substance release.

### Anti-bacterial activity

In order to instead demonstrate the anti-bacterial effectiveness of the composition of the present invention, *in vitro* essays are suitable such as broth dilution (by calculating the MIC minimum inhibitory concentration and the MBC minimum bactericidal concentration) and agar diffusion (where a standard concentration of the specimen is applied into a bacteria broth-culture and the diffusivity of the specimen inside the ground is calculated).

### In vivo test

An experimental model proposes testing the present composition for its antioxidant activity towards oral mucosa cells, obtained from mice palatal tissue. After sacrificing animals, the palatal tissue is aseptically removed and washed with a 1% phosphate buffered saline. The tissue is sectioned into small pieces and digested with 0.25% collagenase for 12 hours. Treatment of the cell cultures with 10 µM of H2O2 leads to a substantial increase of glutathione oxidised forms (GSSG) from 20% to 60%. The object of the model is to verify that by treating cell cultures with the association of N-acetylcysteine, Vitamin C and hyaluronic acid a significant increase occurs in the reduced glutathione form if compared to treatment with H2O2 and treatment with the single substances, proving the antioxidant effectiveness of the composition of the present invention.

### EXAMPLES

Some examples of daily doses of the active components of the compositions object of the present invention will now be provided for illustrative purposes. Daily doses are intended to be administered in a suitable form of oral or topic dosage and subdivided into one or more dosage unit such as a pastille, a candy, a capsule, a tablet or a sachet, a cream, etcetera.

Changes or variations of the herein exemplified embodiments, apparent to a person skilled in the art, are comprised in the enclosed claims.

### EXAMPLE 1

| **Active ingredient** | **Amount for single dosage unit** |
|---|---|
| N-acetylcysteine | 5 mg |
| Vitamin C | 50 mg |
| Hyaluronic acid | 10 mg |

| | |
|---|---|
| Pharmaceutical form: candy | |

### EXAMPLE 2

| **Active ingredient** | **Amount for single dosage unit** |
|---|---|
| N-acetylcysteine | 20 mg |
| Vitamin C | 30 mg |
| Hyaluronic acid | 5 mg |

| | |
|---|---|
| Pharmaceutical form: pastille | |

### EXAMPLE 3

| **Active ingredient** | **Amount for single dosage unit** |
|---|---|
| N-acetylcysteine | 15 mg |
| Vitamin C | 40 mg |
| Hyaluronic acid | 5 mg |

| | |
|---|---|
| Pharmaceutical form: mouth-dissolving tablet | |

### EXAMPLE 4

| **Active ingredient** | **Amount** |
|---|---|
| N-acetylcysteine | 400 mg |
| Vitamin C | 200 mg |
| Hyaluronic acid | 150 mg |

| | |
|---|---|
| Pharmaceutical form: oral bottle | |

### EXAMPLE 5

| **Active ingredient** | **Amount** |
|---|---|
| N-acetylcysteine | 100 mg |
| Vitamin C | 50 mg |
| Hyaluronic acid | 25 mg |

| | |
|---|---|
| Pharmaceutical form: oropharyngeal spray | |

## Claims

1. . A composition comprising an association of N-acetylcysteine (NAC), hyaluronic acid and/or its salts and Vitamin C for use in the prevention and/or treatment of oropharyngeal mucose diseases, , wherein the dosage form comprises N-acetylcysteine (NAC) in an amount between 0.5 mg and 9000 mg, hyaluronic acid and/or its salts in an amount between 0.5 mg and 5000 mg and Vitamin C in amount between 0.5 mg and 5000 mg..

2. . Composition for use according to claim 1, in the form of a solid, semisolid or liquid composition for oral or topic use.

3. . Composition for use according to claims 1 or 2, in the form of pastilles, candies, powder, mounth dissolving powder, granulate, hard capsule, soft-gel capsule, tablet, mounth dissolving tablet, chewable tablet, sachet, solution, suspension, emulsion, spray.

4. . Composition for use according to any of claims from 1 to 3, wherein the dosage form comprises preferably N-acetylcysteine (NAC) in an amount between 1 mg and 7000 mg, still more preferably between 5 mg and 6000 mg; comprises hyaluronic acid and/or its salts preferably in an amount between 1 mg and 3000 mg, still more preferably between 5 mg and 2000 mg and comprises Vitamin C preferably in amount between 1 mg and 3000 mg, still more preferably between 5 mg and 2000 mg.

5. . Composition for use according to any of claims from 1 to 4, wherein is present at least a component selected from the group consisting of honey, propolis, Adhatoda vasica, Grindelia robusta and their combinations.

6. . Composition for use according to any of claims from 1 to 5, for use in the prevention and / or treatment of diseases such as pharyngitis, faringotonsillitis, coughing, dysphonia, hoarseness, epiglottitis, laryngitis, mouth ulcers, mucositis, leukoplakia.

## Patentansprüche

1. Zusammensetzung, umfassend eine Assoziation von N-Acetylcystein (NAC), Hyaluronsäure und/oder deren Salze und Vitamin C zur Verwendung bei der Vorbeugung und/oder Behandlung von oropharyngealen Schleimhauterkrankungen, wobei die Dosierungsform N-Acetylcystein (NAC) in einer Menge zwischen 0,5 mg und 9000 mg, Hyaluronsäure und/oder deren Salze in einer Menge zwischen 0,5 mg und 5000 mg und Vitamin C in einer Menge zwischen 0,5 mg und 5000 mg umfasst.

2. Zusammensetzung zur Verwendung nach Anspruch 1 in Form einer festen, halbfesten oder flüssigen Zusammensetzung zur oralen oder topischen Anwendung.

3. Zusammensetzung zur Verwendung nach Anspruch 1 oder 2, in Form von Pastillen, Bonbons, Pulver, im Mund auflösendes Pulver, Granulat, Hartkapsel, Weichgelkapsel, Tablette, im Mund auflösende Tablette, Kautablette, Beutel, Lösung, Suspension, Emulsion, Spray.

4. Zusammensetzung zur Verwendung nach einem der Ansprüche 1 bis 3, wobei die Dosierungsform vorzugsweise N-Acetylcystein (NAC) in einer Menge zwischen 1 mg und 7000 mg, noch stärker bevorzugt zwischen 5 mg und 6000 mg umfasst; Hyaluronsäure und/oder deren Salze vorzugsweise in einer Menge zwischen 1 mg und 3000 mg, noch stärker bevorzugt zwischen 5 mg und 2000 mg umfasst und Vitamin C vorzugsweise in einer Menge zwischen 1 mg und 3000 mg, noch stärker bevorzugt zwischen 5 mg und 2000 mg umfasst.

5. Zusammensetzung zur Verwendung nach einem der Ansprüche 1 bis 4, wobei mindestens eine Komponente ausgewählt aus der Gruppe bestehend aus Honig, Propolis, Adhatoda vasica, Grindelia robusta und deren Kombinationen zugegen ist.

6. Zusammensetzung zur Verwendung nach einem der Ansprüche 1 bis 5 zur Verwendung bei der Vorbeugung und/oder Behandlung von Krankheiten wie Pharyngitis, Pharyngotonsillitis, Husten, Dysphonie, Heiserkeit, Epiglottitis, Laryngitis, Mundgeschwüren, Mukositis, Leukoplakie.

## Revendications

1. Composition comprenant une association de N-acétylcystéine (NAC), d'acide hyaluronique et/ou de ses sels, et de vitamine C, pour une utilisation dans la prévention et/ou le traitement de maladies de la muqueuse oropharyngée, dans laquelle la forme posologique comprend de la N-acétylcystéine (NAC) en une quantité comprise entre 0,5 mg et 9000 mg, de l'acide hyaluronique et/ou ses sels en une quantité comprise entre 0,5 mg et 5000 mg, et de la vitamine C en une quantité comprise entre 0,5 mg et 5000 mg.

2. Composition pour une utilisation selon la revendication 1, sous la forme d'une composition solide, semi-solide ou liquide à usage oral ou topique.

3. Composition pour une utilisation selon la revendication 1 ou 2, sous la forme de pastilles, bonbons, poudre, poudre se dissolvant dans la bouche, granulat, capsule à enveloppe dure, capsule à enveloppe molle, comprimé, comprimé se dissolvant dans la bouche, comprimé à mâcher, sachet, solution, suspension, émulsion, spray.

4. Composition pour une utilisation selon l'une quelconque des revendications 1 à 3, dans laquelle la forme posologique comprend de la N-acétylcystéine (NAC) de préférence en une quantité comprise entre 1 mg et 7000 mg, mieux encore entre 5 mg et 6000 mg ; comprend de l'acide hyaluronique et/ou ses sels de préférence en une quantité comprise entre 1 mg et 3000 mg, mieux encore entre 5 mg et 2000 mg, et comprend de la vitamine C de préférence en une quantité comprise entre 1 mg et 3000 mg, mieux encore entre 5 mg et 2000 mg.

5. Composition pour une utilisation selon l'une quelconque des revendications 1 à 4, dans laquelle est présent au moins un composé choisi dans le groupe constitué par le miel, la propolis, Adhatoda vasica, Grindelia robusta et leurs combinaisons.

6. Composition pour une utilisation selon l'une quelconque des revendications 1 à 5, pour une utilisation dans la prévention et/ou le traitement de maladies telles qu'une pharyngite, une pharyngo-amygdalite, une toux, une dysphonie, un enrouement, une épiglottite, une laryngite, des ulcères buccaux, une mucosité, une leucoplasie.
